# FASCICULE DE BREVET EUROPEEN

(11) **EP 0 809 516 B1**
(45) Date de publication et mention de la délivrance du brevet: **22.08.2001**
(21) Numéro de dépôt: 96903080.8
(22) Date de dépôt: 12.02.1996
(51) Int. Cl.: A61K 48/00, C12N 15/86

(54) **ASSOCIATION MEDICAMENTEUSE UTILE POUR LA TRANSFECTION ET L'EXPRESSION IN VIVO D'EXOGENES**
MEDIZINISCHE ZUSAMMENSETZUNG ZUR VERWENDUNG FÜR DIE TRANSFEKTION UND IN VIVOEXPRESSION VON EXOGENEN
MEDICINAL COMBINATION USEFUL FOR IN VIVO EXOGENIC TRANSFECTION AND EXPRESSION

(30) Priorité: 14.02.1995 FR 9501662
(43) Date de publication de la demande: 03.12.1997
(73) Titulaire: Aventis Pharma S.A., 92160 Antony (FR); INSTITUT NATIONAL DE LA SANTE ET DE LA RECHERCHE MEDICALE (INSERM), 75013 Paris (FR)
(72) Inventeur: BACH, Jean-François, F-75007 Paris (FR); CHATENOUD, Lucienne, F-92800 Puteaux (FR); HADDADA, Hedi, F-94140 Alfortville (FR); LEE, Martin, F-75010 Paris (FR); PERRICAUDET, Michel, F-28320 Ecrosnes (FR); WEBB, Michelle, F-75005 Paris (FR)
(74) Mandataire: Le Pennec, Magali
(86) Numéro de dépôt international: FR9600218
(87) Numéro de publication internationale: WO9625177

(56) Documents cités:
- WO-A-94/16065
- WO-A-94/16080
- WO-A-94/28152
- BRITISH MEDICAL BULLETIN, vol. 51, no. 1, EDINBURGH, GB, pages 31-44, XP002003907 E. KREMER ET AL.: "Adenovirus and adeno-associated virus mediated gene transfer."
- JOURNAL OF CELLULAR BIOCHEMISTRY, SUPPLEMENT, vol. 0, no. 21A, 31 Mars 1995, NEW YORK, NY, USA, page 359 XP002003908 M. LEE ET AL.: "Reducing the immune response against adenoviral vectors."
- GENE THERAPY, vol. 2 , no. 4 , Juin 1995, BASINGSTOKE, GB, pages 256-262, XP000571413 M. LEE ET AL.: "The constitutive expression of the immunomodulatory gp19K protein in E1-, E3-adenoviral vectors strongly reduces the host cytotoxic T cell response against the vector."

## Description

La présente invention concerne le domaine de la thérapie génique et notamment l'utilisation d'adénovirus pour l'expression de gène d'intérêt thérapeutique. Elle concerne plus particulièrement un nouveau mode de traitement des pathologies d'origine génétique basé sur l'utilisation combinée de deux types d'agents thérapeutiques.

La thérapie génique consiste à corriger une déficience ou une anomalie (mutation, expression aberrante, etc) par introduction d'une information génétique dans la cellule ou l'organe affecté. Cette information génétique peut être introduite soit in vitro ou ex vivo dans une cellule extraite de l'organe, la cellule modifiée étant alors réintroduite dans l'organisme, soit directement in vivo dans le tissu approprié. Dans ce second cas, différentes techniques physiques de transfection existent, parmi lesquelles l'emploi de virus comme vecteurs. A cet égard, différents virus ont été testés pour leur capacité à infecter certaines populations cellulaires. En particulier, les rétrovirus (RSV, HMS, MMS, etc), le virus HSV, les virus adéno-associés, et les adénovirus.

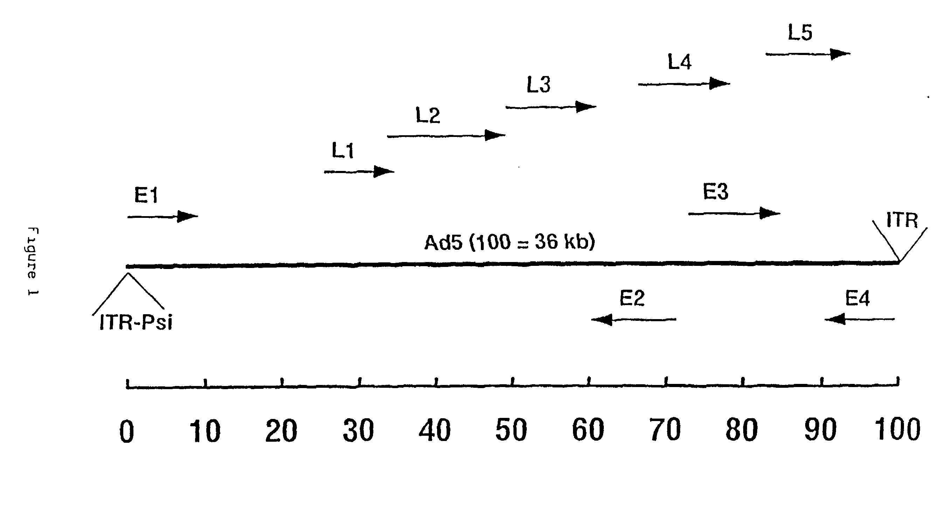

Parmi ces virus, les adénovirus présentent certaines propriétés intéressantes pour une utilisation en thérapie génique. Ils ont un spectre d'hôte assez large, sont capables d'infecter des cellules quiescentes et ne s'intègrent pas au génome de la cellule infectée. Les adénovirus sont des virus à ADN double brin linéaire d'une taille de 36 kb environ. Leur génome comprend notamment une séquence inversée répétée (ITR) à leur extrémité, une séquence d'encapsidation, des gènes précoces et des gènes tardifs (Cf figure 1). Les principaux gènes précoces sont les gènes E1 (E1a et E1b), E2, E3 et E4. Les principaux gènes tardifs sont les gènes L1 à L5.

Compte tenu des propriétés des adénovirus mentionnées ci-dessus, ceux-ci ont déjà été utilisés pour le transfert de gènes in vivo. A cet effet, différents vecteurs dérivés des adénovirus ont été préparés, incorporant différents gènes (β-gal, OTC, α-1AT, cytokines, etc). Dans chacune de ces constructions, l'adénovirus a été modifié de manière à le rendre incapable de réplication dans la cellule infectée. Ainsi, les constructions décrites dans l'art antérieur sont des adénovirus délétés des régions E1 (E1a et/ou E1b) et éventuellement E3 au niveau desquelles est insérée une séquence d'ADN hétérologue (Levrero et al., Gene 101 (1991) 195; Gosh-Choudhury et al., Gene 50 (1986) 161).

Cependant, comme pour tous les virus connus, l'administration d'un adénovirus sauvage (Routes et al., J. Virol. 65 (1991) 1450) ou recombinant défectif pour la réplication (Yang et al., PNAS (1994) 4407), induit une réponse immunitaire importante.

La finalité première du système immunitaire est l'intégrité de l'individu ou l'intégrité du « soi ». Elle aboutit à l'élimination des agents infectieux ainsi qu'au rejet des greffes et des tumeurs sans toutefois que ces puissants mécanismes de défense de l'organisme ne se retournent contre lui et ne donnent lieu à des maladies autoimmunes. Cet état de non-réponse vis-à-vis des antigènes de « soi » alors que les antigènes étrangers sont éliminés est défini comme un état de tolérance physiologique. Afin d'éliminer les agents étrangers, le système immunitaire développe deux types de mécanismes. Le premier est la production d'anticorps spécifiques par les lymphocytes B ; on parle d'immunité humorale. Ces anticorps vont fixer l'antigène et soit l'inactiver soit l'éliminer de l'organisme. Le second mécanisme de défense concerne l'immunité cellulaire et met en oeuvre des lymphocytes T, et parmi ceux-ci des lymphocytes T cytotoxiques, porteurs d'un récepteur spécifique de l'antigène considéré. La reconnaissance de l'antigène par le récepteur T nécessite que celui-ci soit exprimé en association avec des protéines codées par les gènes du complexe majeur d'histocompatibilité ou CMH de classe I et de classe II.

En conséquence, cette réponse immunitaire développée contre les cellules infectées constitue un obstable majeur à l'utilisation des vecteurs viraux en thérapie génique puisque (i) en induisant une destruction des cellules infectées elle limite la durée d'expression du gène thérapeutique et donc l'effet thérapeutique, (ii) elle induit parallèlement une réponse inflammatoire importante, et (iii) elle entraîne l'élimination rapide des cellules infectées après des injections répétées. Il est entendu que l'amplitude de cette réponse immunitaire à l'encontre des cellules infectées varié selon la nature de l'organe subissant l'injection et le mode d'injection mis en oeuvre. C'est ainsi que l'expression de la β-galactosidase, codée par un adénovirus recombinant administré dans le muscle de souris immunocompétentes, est réduite à des niveaux minimum 40 jours après l'injection (Kass-Eisler et al., PNAS 90 (1993) 11498). De même, l'expression de gènes transfectés par des adénovirus dans le foie est significativement réduite dans les 10 jours qui suivent l'injection (Yang Y et al. 1994 immunity 1 433-442) et l'expression du facteur IX transféré par adénovirus dans des hépatocytes de chiens hémophiles disparait 100 jours après l'injection (Kay et al., PNAS 91 (1994) 2353).

Dans la perspective d'une exploitation en thérapie génique de vecteurs dérivés des adénovirus, il apparait donc nécessaire de contrôler la réponse immunitaire développée à leur encontre ou contre les cellules qu'ils infectent.

De ce qui précède, il ressort que la mise en activité du système immunitaire nécessite au préalable une reconnaissance par celui-ci des éléments étrangers à l'organisme (le non-soi ou soi modifié) comme des vecteurs dérivés des adenovirus, qui doivent en temps normal être détruits. Au cours des dernières années des stratégies d'immunointervention ont été développées dont le but est de créer un environnement immunitaire « permissif », c'est-à-dire à l'induction d'un état de tolérance, vis-à-vis d'antigènes étrangers pré-définis.

C'est précisément à ce niveau qu'intervient la présente invention. Elle vise à prévenir l'élimination rapide des adénovirus des cellules infectées et donc à prolonger de manière conséquente l'expression in vivo du gène thérapeutique qu'ils portent.

Récemment, les demandeurs ont mis en évidence que la co-expression dans les cellules infectées de certains gènes est capable d'induire un effet immunoprotecteur et ainsi de faire échapper les vecteurs et/ou les cellules infectées au système immunitaire. Ils ont notamment mis au point des adénovirus dans lesquels l'expression d'un gène d'intérêt thérapeutique est couplée à celle d'un gène immunoprotecteur ( FR N°94 12346). Il peut notamment s'agir d'un gène dont le produit agit sur l'activité du complexe majeur d'histocompatibilité (MHC) ou sur l'activité des cytokines permettant de réduire considérablement, voire de supprimer toute réaction immunitaire à l'encontre du vecteur ou des cellules infectées. Ils inhibent au moins partiellement l'expression des protéines MHC ou la présentation antigénique avec pour conséquence avantageuse une réduction notable de la réaction immunitaire à l'encontre du vecteur ou des cellules infectées et donc un effet thérapeutique prolongé.

De manière inattendue, les demandeurs ont mis en évidence qu'il était possible de prolonger significativement l'effet thérapeutique d'un tel vecteur en lui associant un immunosuppresseur. L'élimination du vecteur considéré et/ou la destruction des cellules infectées, par le système immunitaire, se trouvent retardées d'un délai nettement supérieur à celui auquel on pouvait s'attendre par la simple juxtaposition des effets immunoprotecteurs du dit vecteur et de l'immunosuppresseur. Avantageusement, l'association médicamenteuse objet de la présente invention induit un phénomène de « pseudo-inertie » du sytème immunitaire propice à l'expression sur le long terme d'un gène thérapeutique.

Au sens de l'invention, immunosuppresseur désigne tout composé capable d'inhiber en partie ou en totalité au moins une voie de signalisation immunitaire. De manière générale, les immunosuppresseurs sont habituellement utilisés en transplantation, dans le but de prévenir le rejet d'allogreffe, et dans le traitement de certaines maladies autoimmunes. Les produits classiquement utilisés sont soit les immunosuppresseurs chimiques comme les corticostéroïdes, l'azathioprine, la cyclosporine, le FK506, soit des immunosuppresseurs biologiques tels les anticorps polyclonaux ou monoclonaux. La première catégorie d'immunosuppresseurs, et parmi ceux-ci surtout la cyclosporine et le FK506, inhibent de manière importante la production de cytokines, telles l'interleukine 2, dont le rôle dans la différentiation et la prolifération des cellules lymphocytaires est essentiel. Malheusement, l'efficacité de ce type d'immunosuppresseurs nécessite leur administration permanente qui est confrontée à plus ou moins long terme au problème de leur toxicité. Ainsi l'azzathioprine est potentiellement myelotoxique et la cyclosporine est néphrotoxique et peut en outre entraîner une hypertension ou des troubles neurologiques.

En ce qui concerne plus particulièrement les anticorps, il s'agit d'anticorps dirigés contre les cellules lymphoïdes du système immunitaire. Le premier anticorps utilisé à titre d'immunosuppresseur est l'anti-CD3, dirigé contre les lymphocytes T. Sa cible est l'une des chaînes polypeptidiques de la molécule CD3 qui compose le récepteur pour l'antigène des cellules T. Il s'en suit une inactivation fonctionnelle des cellules T CD3+ reconnues par l'anticorps. Dans le cas de la problématique qui nous intéresse ici, l'administration d'un immunosuppresseur de ce type conjointement à celle d'un adénovirus recombinant contenant un gène thérapeutique serait en mesure de bloquer la réaction immunitaire de l'hôte vis-à-vis du vecteur viral et/ou de ses produits exprimés à la surface des cellules infectées. Sur le même principe on peut faire usage d'anticorps anti-CD4, -CD2, -CD8, -CD28, -B7, -ICAM-1 et -LFA-1.

Les demandeurs ont a maintenant mis au point un nouveau mode de traitement particulièrement efficace pour retarder de manière importante voire inhiber la réaction du système immunitaire sans soulever de problèmes de toxicité.
Plus précisément, la présente invention découle de la mise en évidence d'un effet synergique particulièrement important lié à l'utilisation combinée d'un adénovirus recombinant dans lequel l'expression d'un gène d'intérêt thérapeutique est couplée à celle d'un gène immunoprotecteur, tel que décrit précédemment, et au moins un agent immunosuppresseur.

Un premier objet de la présente invention concerne donc une association médicamenteuse d'au moins un agent immunosuppresseur et d'au moins un adénovirus recombinant dont le génome comprend un premier ADN recombinant contenant un gène thérapeutique et un second ADN recombinant contenant un gène immunoprotecteur, pour une utilisation consécutive, intermittente et/ou simultanée dans le temps, utile pour des transfections exogéniques in vivo et/ou ex-vivo.
Comme indiqué ci-avant, l'invention repose notamment sur la mise en évidence d'un effet synergique entre l'activité de l'agent immunosuppresseur et l'effet du gène immunoprotecteur exprimé sur l'expression du gène thérapeutique.
Cette utilisation combinée permet un effet thérapeutique nettement prolongé et nécessite avantageusement des doses significativement amoindries notamment en agent immunosuppresseur.
Comme indiqué plus loin, les deux composantes de l'association combinées de la présente invention peuvent être utilisées de manière consécutive, intermittente et/ou simultanée dans le temps. De préférence, l'agent immunosuppresseur est injecté avant et après l'injection de l'adénovirus. Selon ce mode de mise en oeuvre de la présente invention, l'administration du l'immunosuppresseur peut être espacée dans le temps et plus préférentiellement renouvellée régulièrement. Dans ce cas particulier les deux composants sont conditionnés séparément. Dans le cas d'une administration simultannée, ils peuvent être mélangés extemporanément avant d'être administrés ensemble ou au contraire administrés simultanément mais de manière séparée. En particulier, les voies d'administration des deux agents peuvent être différentes.

Selon la présente invention, on peut utiliser, à titre d'agent immunosuppresseur, tout composé capable d'inhiber en partie ou en totalité au moins une voie de signalisation immunitaire. Il peut notamment être choisi parmi la cyclosporine, le FK506, l'azathioprine, les corticostéroïdes et tout anticorps mono- ou poly-clonal. Il s'agit de préférence d'anticorps capables d'inactiver les molécules immunes ou de provoquer la destruction des cellules immunes portant ces molécules. On peut notamment utiliser à titre d'anticorps, les anti-CD4, -CD3, CD2, -CD8,-CD28, -B7, -ICAM-1, -LFA-1. Il peut également s'agir de molécules hybrides comme la CTLA4Ig, une protéine de fusion entre la molécule CTLA-4 (un homologue au CD28) et une immunoglobuline. Le site G1Fc de cette molécule en se liant à la molécule B7 s'avère capable d'inhiber l'activation des cellules T ( D. J; Lenschow; Science, 257, 789, 1992). Il est clair que la portée de la présente invention n'est aucunement limitée aux immunosuppresseurs énumérés ci-dessus. Ces immunosuppresseurs peuvent être employés sous forme isolée ou en association.

En ce qui concerne les ADN recombinants présents dans le génome de l'adénovirus mis en oeuvre selon la présente invention, il s'agit de fragments d'ADN contenant le gène considéré (thérapeutique ou immunoprotecteur) et éventuellement des signaux permettant son expression, construits in vitro puis insérés dans le génome de l'adénovirus. Les ADN recombinants utilisés dans le cadre de la présente invention peuvent être des ADN complémentaires (ADNc), des ADN génomiques (ADNg), ou des constructions hybrides consistant par exemple en un ADNc dans lequel seraient insérés un ou plusieurs introns. Il peut également s'agir de séquences synthétiques ou semisynthétiques. Ces ADN peuvent être d'origine humaine, animale, végétale, bactérienne, virale, etc. De manière particulièrement avantageuse, on utilise des ADNc ou des ADNg.

Comme gène thérapeutique utilisable pour la construction des vecteurs de la présente invention, on peut citer tout gène codant pour un produit ayant un effet thérapeutique. Le produit ainsi codé peut être une protéine, un peptide, un ARN, etc.
S'agissant d'un produit protéique, il peut être homologue vis-à-vis de la cellule cible (c'est-à-dire un produit qui est normalement exprimé dans la cellule cible lorsque celle-ci ne présente aucune pathologie). Dans ce cas, l'expression d'une protéine permet par exemple de pallier une expression insuffisante dans la cellule ou l'expression d'une protéine inactive ou faiblement active en raison d'une modification, ou encore de surexprimer ladite protéine. Le gène thérapeutique peut aussi coder pour un mutant d'une protéine cellulaire, ayant une stabilité accrue, une activité modifiée, etc. Le produit protéique peut également être hétérologue vis-à-vis de la cellule cible. Dans ce cas, une protéine exprimée peut par exemple compléter ou apporter une activité déficiente dans la cellule, lui permettant de lutter contre une pathologie, ou stimuler une réponse immunitaire.
Parmi les produits protéiques thérapeutiques au sens de la présente invention, on peut citer plus particulièrement les enzymes, les dérivés sanguins, les hormones, interleukines, interférons, TNF, etc (FR 9203120), les facteurs de croissance, les neurotransmetteurs ou leurs précurseurs ou enzymes de synthèse, les facteurs trophiques : BDNF, CNTF, NGF, IGF, GMF, aFGF, bFGF, NT3, NT5, HARP/pléiotrophine, etc; les apolipoprotéines : ApoAI, ApoAIV, ApoE, etc (FR 93 05125), la dystrophine ou une minidystrophine (FR 9111947), la protéine CFTR associée à la mucoviscidose, les gènes suppresseurs de tumeurs : p53, Rb, Rap1A, DCC, k-rev, etc (FR 93 *04745),* les gènes codant pour des facteurs impliqués dans la coagulation : Facteurs VII, VIII, IX, les gènes intervenant dans la réparation de l'ADN, etc.

Comme indiqué plus haut, le gène thérapeutique peut également être un gène ou une séquence antisens, dont l'expression dans la cellule cible permet de contrôler l'expression de gènes ou la transcription d'ARNm cellulaires. De telles séquences peuvent, par exemple, être transcrites dans la cellule cible en ARN complémentaires d'ARNm cellulaires et bloquer ainsi leur traduction en protéine, selon la technique décrite dans le brevet EP 140 308. Les antisens comprennent également les séquences codant pour des ribozymes, qui sont capables de détruire sélectivement des ARN cibles (EP 321 201).
Les gènes thérapeutiques peuvent être d'origine humaine, animale, végétale, bactérienne, virale, etc. Ils peuvent être obtenus par toute technique connue de l'homme du métier, et notamment par criblage de banques, par synthèse chimique, ou encore par des méthodes mixtes incluant la modification chimique ou enzymatique de séquences obtenues par criblage de banques.

Le gène immunoprotecteur utilisé dans le cadre de la présente invention peut être de différents types. Comme explicité précédemment, il s'agit d'un gène dont le produit agit sur l'activité du complexe majeur d'histocompatibilité (MHC) ou sur l'activité des cytokines Il s'agit de préférence d'un gène dont le produit inhibe au moins partiellement l'expression des protéines du MHC ou la présentation antigénique. A titre d'exemples préférés, on peut citer certains gènes contenus dans la région E3 de l'adénovirus, le gène ICP47 du virus de l'herpès, ou le gène UL18 du cytomégalovirus.

La région E3 du génome de l'adénovirus contient différentes phases de lecture qui, par épissage alternatif, donnent naissance à des protéines différentes. Parmi celles-ci, la protéine Gp19k (ou E3-19k) est une protéine transmembranaire glycosylée localisée dans la membrane du réticulum endoplasmique (RE). Cette protéine comprend un domaine luminal liant les molécules du MHC-I et une extrémité cytoplasmique C-terminale capable de lier les microtubules (ou la tubuline), qui agit pour ancrer la protéine gp19k dans la membrane du RE. Gp19k est ainsi capable d'empêcher l'expression des molécules MHC-I à la surface des cellules par interaction et séquestration au niveau du RE. Cependant, en l'absence de réplication virale, la protéine gp19k est faiblement exprimée par les adénovirus. Par ailleurs, l'expression de la gp19k est également conditionnée à la réalisation d'un épissage. L'introduction dans les vecteurs de l'invention d'un ADN recombinant contenant une séquence (ADNc de préférence) codant pour la gp19k permet de contrôler et d'optimiser l'expression de ladite protéine. En particulier, l'emploi de promoteurs constitutifs et la suppression des autres phases de lecture permet d'augmenter fortement l'expression de cette protéine et de s'affranchir de la dépendance vis-à-vis de la réplication virale et la présence d'éléments inducteurs. Ceci permet de manière particulièrement avantageuse de diminuer considérablement la lyse par les CTL des cellules infectées et ainsi d'augmenter et de prolonger la production in vivo du gène thérapeutique.

D'autres protéines codées par la région E3 du génome de l'adénovirus telles que les protéines 10,4k et 14,5k présentent certaines propriétés intéressantes en vue de leur incorporation dans les vecteurs de l'invention.

Le gène ICP47 du virus de l'herpès simplex constitue un autre gène immunoprotecteur particulièrement intéressant au sens de la présente invention. Les cellules infectées par le virus de l'herpès simplex présentent une résistance à la lyse induite par les CTL. Il a été montré que cette résistance pouvait être conférée par le gène ICP47, qui est capable de réduire l'expression des molécules MHC-I à la surface des cellules. L'incorporation du gène ICP47 dans un ADN recombinant selon l'invention permet également aux virus recombinants de l'invention d'échapper au système immunitaire.

Le gène UL18 du cytomégalovirus constitue un autre exemple préféré de gène immunoprotecteur selon l'invention. Le produit du gène UL18 est capable de lier la β2-microglobuline (Browne et al. Nature 347 (1990) 770). La β2-microglobuline est l'une des chaines des molécules MHC-I. L'incorporation du gène UL18 dans un ADN recombinant selon l'invention permet ainsi de diminuer le nombre de molécules de β2-microglobuline fonctionnelles dans les cellules infectées par les virus de l'invention, et donc de diminuer les capacités de ces cellules à produire des molécules MHC-I complètes et fonctionnelles. Ce type de construction permet donc de protéger les cellules infectées de la lyse par les CTL.

Comme indiqué ci-avant, le gène immunoprotecteur utilisé dans le cadre de la présente invention est, dans un autre mode de réalisation préféré, un gène dont le produit inhibe l'activité ou les voies de signalisation des cytokines. Les cytokines constituent une famille de protéines sécrétées qui agissent comme des molécules de signalisation pour le système immunitaire. Elles peuvent attirer les cellules de l'immunité, les activer, induire leur prolifération et même agir directement sur les cellules infectées pour les tuer.

Parmi les gènes dont le produit affecte sur l'activité ou les voies de signalisation des cytokines, on peut citer les gènes intervenant sur la synthèse des cytokines, ou dont le produit est capable de séquestrer les cytokines, d'antagoniser leur activité ou d'interférer avec les voies de signalisation intercellulaires. A titre d'exemple préférentiels, on peut citer en particulier le gène BCRF1 du virus d'Epstein Barr, les gènes crmA et crmB du virus de cowpox, les gènes B15R et B18R du virus de la vaccine, le gène US28 du cytomégalovirus, les gènes E3-14,7, E3-10,4 et E3-14,5 de l'adénovirus.

Le gène B15R du virus de la vaccine code pour une protéine soluble capable de lier l'interleukine-1β (la forme sécrétée de l'interleukine-1), et ainsi d'empêcher cette cytokine de se lier à ses récepteurs cellulaires. L'interleukine-1 est en effet l'une des premières cytokines produites en réponse à une agression antigénique, et elle joue un role très important dans la signalisation du système immunitaire au début de l'infection. La possibilité d'incorporer le gène B15R dans un vecteur selon l'invention permet avantageusement de réduire l'activité de l'IL-1β, notamment sur l'activation des cellules immunitaires, et de ce fait de protéger localement les cellules infectées par les virus de l'invention contre une réponse immunitaire importante. Des gènes homologues au gène B15R peuvent également être utilisés, tel que le gène du virus de cowpox.

De la même manière, le gène B18R du virus de la vaccine code pour une protéine homologue au récepteur de l'interleukine-6. Ce gène, ou tout homologue fonctionnel, est également utilisable dans les vecteurs de l'invention pour inhiber la liaison de l'interleukine-6 sur son récepteur cellulaire et ainsi réduire localement la réponse immunitaire.
Toujours de la même façon, le gène crmB du virus de cowpox peut être avantageusement utilisé. Ce gène code en effet pour une protéine sécrétée capable de lier le TNF et d'entrer en compétition avec les récepteurs du TNF à la surface des cellules. Ce gène permet donc, dans les virus de l'invention, de diminuer localement la concentration de TNF actif susceptible de détruire les cellules infectées. D'autres gènes codant pour des protéines capables de lier le TNF et d'inhiber au moins partiellement sa liaison sur ses récepteurs peuvent également être utilisés.
Le gène crmA du virus de cowpox code lui pour une protéine ayant une activité d'inhibiteur de protéases du type serpine, qui est capable d'inhiber la synthèse de l'interleukine-1β. Ce gène peut donc être utilisé pour diminuer localement la concentration en interleukine-1 et ainsi réduire le développement de la réponse immunitaire et inflammatoire.
Le gène BCRF1 du virus d'Epstein Barr code pour un analogue de l'interleukine 10. Le produit de ce gène est une cytokine capable de diminuer la réponse immunitaire et de changer sa spécificité, tout en induisant la prolifération des lymphocytes B.
Le gène US28 du cytomégalovirus code pour une protéine homologue au récepteur de la protéine inflammatoire des macrophages 1α (MIP-1α). Cette protéine est donc capable d'agir comme compétiteur des récepteurs du MIP, et donc d'inhiber son activité localement.
Le produit des gènes E3-14,7, E3-10,4 et E3-14,5 de l'adénovirus est capable de bloquer la transmission du signal intercellulaire médié par certaines cytokines. Lorsque les cytokines se lient à leur récepteur à la surface d'une cellule infectée, un signal est transmis au noyau pour induire la mort cellulaire ou stoper la synthèse protéique. C'est le cas en particulier du facteur de nécrose des tumeurs (TNF). L'incorporation des gènes E3-14,7, E3-10,4 et/ou E3-14,5 dans un ADN recombinant selon l'invention en vue de leur expression constitutive ou régulée permet de bloquer la signalisation intercellulaire induite par le TNF, et ainsi de protéger les cellules infectées par les virus recombinants de l'invention des effets toxiques de cette cytokine.

Une inhibition locale et transitoire peut être particulièrement avantageuse. Celle-ci peut être obtenue notamment par le choix des signaux d'expression particuliers (promoteurs cytokine-dépendants par exemple) comme indiqué ci-après.

Il est entendu que d'autres gènes homologues ou ayant des propriétés fonctionnelles similaires peuvent être utilisés pour la construction des vecteurs de l'invention. Ces différents gènes peuvent être obtenus par toute technique connue de l'homme du métier, et notamment par criblage de banques, par synthèse chimique, ou encore par des méthodes mixtes incluant la modification chimique ou enzymatique de séquences obtenues par criblage de banques. En outre, ces différents gènes peuvent être utilisés seuls ou en combinaison(s).

L'insertion des gènes considérés sous forme d'ADN recombinants selon l'invention offre une plus grande flexibilité dans la construction des adénovirus, et permet un meilleur contrôle de l'expression desdits gènes.

Ainsi, les ADN recombinants (et donc les deux gènes d'intérêt) incorporés dans les vecteurs adénoviraux selon la présente invention peuvent être agencés de différentes manières.

Ils peuvent tout d'abord être insérés en un même site du génome de l'adénovirus, ou en des sites différents, sélectionnés. En particulier, les ADN recombinants peuvent être insérés au moins en partie au niveau des régions E1, E3 et/ou E4 du génome de l'adénovirus, en remplacement ou en supplément de séquences virales.

Préférentiellement, les ADN recombinants sont insérés, au moins en partie, au niveau des régions E1, E3 ou E4 du génome de l'adénovirus. Lorsqu'ils sont insérés en deux sites différents, on préfère dans le cadre de l'invention utiliser les régions E1 et E3 ou E1 et E4. Les exemples montrent en effet que cette organisation permet une expression élevée des deux gènes, sans interférence entre les deux. Avantageusement, les ADN recombinants sont insérés en remplacement de séquences virales.

Ces ADN recombinants peuvent ensuite comporter chacun un promoteur transcriptionnel, identique ou différent. Cette configuration permet d'obtenir des niveaux d'expression supérieurs, et offre un meilleur contrôle de l'expression des gènes. Dans ce cas, les deux gènes peuvent être insérés dans la même orientation ou dans les orientations opposées.

Ils peuvent également constituer une entité transcriptionnelle unique. Dans cette configuration, les deux ADN recombinants sont contigus et positionnés de telle sorte que les deux gènes soient sous le contrôle d'un promoteur unique, et donnent lieu à un ARN prémessager unique. Cette disposition est avantageuse puisqu'elle permet d'utiliser un seul promoteur transcriptionnel.
Enfin, l'emploi d'ADN recombinants selon l'invention permet d'utiliser des promoteurs transcriptionnels de nature différente, et notamment des promoteurs forts ou faibles, régulés ou constitutifs, tissu-spécifiques ou ubiquitaires, etc.
Le choix des signaux d'expression et de la position respective des ADN recombinants est particulièrement important pour obtenir une expression élevée du gène thérapeutique et un effet immunoprotecteur important.

Un mode particulièrement préféré de mise en oeuvre de la présente invention met en oeuvre un adénovirus défectif comportant un premier ADN recombinant contenant un gène thérapeutique et un second ADN recombinant contenant un gène immunoprotecteur, dans lequel les deux ADN recombinants sont insérés au niveau de la région E1.

Un mode particulièrement préféré de mise en oeuvre de la présente invention met en oeuvre un adénovirus défectif comportant un premier ADN recombinant contenant un gène thérapeutique, inséré au niveau de la région E1,et un second ADN recombinant contenant un gène immunoprotecteur, inséré au niveau de la région E3.

Comme indiqué ci-avant, les adénovirus de la présente invention sont défectifs, c'est-à-dire qu'ils sont incapables de se répliquer de façon autonome dans la cellule cible. Généralement, le génome des adénovirus défectifs selon la présente invention est donc dépourvu au moins des séquences nécessaires à la réplication dudit virus dans la cellule infectée. Ces régions peuvent être soit éliminées (en tout ou en partie), soit rendues non-fonctionnelles, soit substituées par d'autres séquences et notamment par les gènes thérapeutiques. Le caractère défectif des adénovirus de l'invention est un élément important, puisqu'il assure la non dissémination des vecteurs de l'invention après administration.
Dans un mode de réalisation préféré, les adénovirus de l'invention comprennent les séquences ITR et une séquence permettant l'encapsidation, et possèdent une délétion de tout ou partie du gène E1.

Les séquences inversées répétées (ITR) constituent l'origine de réplication des adénovirus. Elles sont localisées aux extrémités 3' et 5' du génome viral (Cf figure 1), d'où elles peuvent être isolées aisément selon les techniques classiques de biologie moléculaire connues de l'homme du métier. La séquence nucléotidique des séquences ITR des adénovirus humains (en particulier des sérotypes Ad2 et Ad5) est décrite dans la littérature, ainsi que des adénovirus canins (notamment CAV1 et CAV2). Concernant l'adénovirus Ad5 par exemple, la séquence ITR gauche correspond à la région comprenant les nucléotides 1 à 103 du génome.
La séquence d'encapsidation (également désignée séquence Psi) est nécessaire à l'encapsidation de l'ADN viral. Cette région doit donc être présente pour permettre la préparation d'adénovirus recombinants défectifs selon l'invention. La séquence d'encapsidation est localisée dans le génome des adénovirus, entre l'ITR gauche (5'). et le gène E1 (Cf figure 1). Elle peut être isolée ou synthétisée artificiellement par les techniques classiques de biologie moléculaire. La séquence nucléotidiques de la séquence d'encapsidation des adénovirus humains (en particulier des sérotypes Ad2 et Ad5) est décrite dans la littérature, ainsi que des adénovirus canins (notamment CAV1 et CAV2). Concernant l'adénovirus Ad5 par exemple, la séquence d'encapsidation correspond à la région comprenant les nucléotides 194 à 358 du génome.

Plus préférentiellement, les adénovirus de l'invention comprennent les séquences ITR et une séquence permettant l'encapsidation, et possèdent une délétion de tout ou partie des gènes E1 et E4.

Dans un mode particulièrement préféré de réalisation, le génome des adénovirus selon l'invention est délété de tout ou partie des gènes E1, E3 et E4, et, encore plus préférentiellement, de tout ou partie des gènes E1, E3, L5 et E4.

Les adénovirus de l'invention peuvent être préparés à partir d'adénovirus d'origines diverses. Il existe en effet différents sérotypes d'adénovirus, dont la structure et les propriétés varient quelque peu, mais qui présentent une organisation génétique comparable. Ainsi, les enseignements décrits dans la présente demande peuvent être aisément reproduits par l'homme du métier pour tout type d'adénovirus.

Plus particulièrement, les adénovirus de l'invention peuvent être d'origine humaine, animale, ou mixte (humaine et animale).

Concernant les adénovirus d'origine humaine, on préfère utiliser ceux classés dans le groupe C. Plus préférentiellement, parmi les différents sérotypes d'adénovirus humain, on préfère utiliser dans le cadre de la présente invention les adénovirus de type 2 ou 5 (Ad 2 ou Ad 5).

Comme indiqué plus haut, les adénovirus de l'invention peuvent également être d'origine animale, ou comporter des séquences issues d'adénovirus d'origine animale. La demanderesse a en effet montré que les adénovirus d'origine animale sont capables d'infecter avec une grande efficacité les cellules humaines, et qu'ils sont incapables de se propager dans les cellules humaines dans lesquelles ils ont été testés (Cf demande FR 93 05954). La demanderesse a également montré que les adénovirus d'origine animale ne sont nullement trans-complémentés par des adénovirus d'origine humaine, ce qui élimine tout risque de recombinaison et de propagation in vivo, en présence d'un adénovirus humain, pouvant conduire à la formation d'une particule infectieuse. L'utilisation d'adénovirus ou de régions d'adénovirus d'origine animale est donc particulièrement avantageuse puisque les risques inhérents à l'utilisation de virus comme vecteurs en thérapie génique sont encore plus faibles.

Les adénovirus d'origine animale utilisables dans le cadre de la présente invention peuvent être d'origine canine, bovine, murine, (exemple : Mav1, Beard et al., Virology 75 (1990) 81), ovine, porcine, aviaire ou encore simienne (exemple : SAV). Plus particulièrement, parmi les adénovirus aviaires, on peut citer les sérotypes 1 à 10 accessibles à l'ATCC, comme par exemple les souches Phelps (ATCC VR-432), Fontes (ATCC VR-280), P7-A (ATCC VR-827), IBH-2A (ATCC VR-828), J2-A (ATCC VR-829), T8-A (ATCC VR-830), K-11 (ATCC VR-921) ou encore les souches référencées ATCC VR-831 à 835. Parmi les adénovirus bovins, on peut utiliser les différents sérotypes connus, et notamment ceux disponibles à l'ATCC (types 1 à 8) sous les référencesATCC VR-313, 314, 639-642, 768 et 769. On peut également citer les adénovirus murins FL (ATCC VR-550) et E20308 (ATCC VR-528), l'adénovirus ovin type 5 (ATCC VR-1343), ou type 6 (ATCC VR-1340); l'adénovirus porcin 5359), ou les adénovirus simiens tels que notamment les adénovirus référencée à l'ATCC sous les numéros VR-591-594, 941-943, 195-203, etc.

De préférence, parmi les différents adénovirus d'origine animale, on utilise dans le cadre de l'invention des adénovirus ou des régions d'adénovirus d'origine canine, et notamment toutes les souches des adénovirus CAV2 [souche manhattan ou A26/61 (ATCC VR-800) par exemple]. Les adénovirus canins ont fait l'objet de nombreuses études structurales. Ainsi, des cartes de restriction complètes des adénovirus CAV1 et CAV2 ont été décrites dans l'art antérieur (Spibey et al., J. Gen. Virol. 70 (1989) 165), et les gènes E1a, E3 ainsi que les séquences ITR ont été clonés et séquencés (voir notamment Spibey et al., Virus Res. 14 (1989) 241; Linné, Virus Res. 23 (1992) 119, WO 91/11525).

Les adénovirus recombinants défectifs selon l'invention peuvent être préparés de différentes façons.
Une première méthode consiste à transfecter l'ADN du virus recombinant défectif préparé in vitro (soit par ligature, soit sous forme de plasmide) dans une lignée cellulaire compétente, c'est-à-dire portant en trans toutes les fonctions nécessaires à la complémentation du virus défectif. Ces fonctions sont préférentiellement intégrées dans le génome de la cellule, ce qui permet d'éviter les risques de recombinaison, et confère une stabilité accrue à la lignée cellulaire.
Une seconde approche consiste à co-transfecter dans une lignée cellulaire apppropriée l'ADN du virus recombinant défectif préparé in vitro (soit par ligature, soit sous forme de plasmide) et l'ADN d'un virus helper. Selon cette méthode, il n'est pas nécessaire de disposer d'une lignée cellulaire compétente capable de complémenter toutes les fonctions défectives de l'adénovirus recombinant. Une partie de ces fonctions est en effet complémentée par le virus helper. Ce virus helper doit lui-même être défectif et la lignée cellulaire porte en trans les fonctions nécessaires à sa complémentation. Parmi les lignées cellulaires utilisables notamment dans le cadre de cette seconde approche, on peut citer notamment la lignée de rein embryonnaire humain 293, les cellules KB, les cellules Hela, MDCK, GHK, etc (Cf exemples).
Ensuite, les vecteurs qui se sont multipliés sont récupérés, purifiés et amplifiés selon les techniques classiques de biologie moléculaire.

Selon une variante de mise en oeuvre, il est possible de préparer in vitro, soit par ligature, soit sous forme de plasmide, l'ADN du virus recombinant défectif portant les délétions appropriées et les deux ADN recombinants. Comme indiqué ci-avant, les vecteurs de l'invention possèdent avantageusement une délétion de tout ou partie de certains gènes viraux, notammment des gènes E1, E3, E4 et/ou L5. Cette délétion peut correspondre à tout type de suppression affectant le gène considéré, Il peut s'agir notamment de la suppression de tout ou partie de la région codante dudit gène, et/ou de tout ou partie de la région promotrice de la transcription dudit gène. La suppression est généralement réalisée sur l'ADN du virus recombinant défectif, par exemple par digestion au moyen d'enzymes de restriction appropriées, puis ligature, selon les techniques de biologie moléculaire, ainsi qu'illustré dans les exemples. Les ADN recombinants peuvent ensuite être insérés dans cet ADN par clivage enzymatique puis ligature, au niveau des régions sélectionnées et dans l'orientation choisie.
L'ADN ainsi obtenu, qui porte donc les délétions appropriées et les deux ADN recombinants, permet de générer directement l'adénovirus recombinant défectif portant lesdites délétions et ADN recombinants. Cette première variante est particulièrement adaptée à la réalisation d'adénovirus recombinants dans lesquels les gènes sont disposés sous forme d'une unité transcriptionnelle unique ou, sous contrôle de promoteurs séparés mais insérés en un même site du génome.

Il est également possible de préparer le virus recombinant en deux étapes, permettant l'introduction successive des deux ADN recombinants. Ainsi, l'ADN d'un premier virus recombinant portant les délétions appropriées (ou une partie desdites délétions) et un des ADN recombinants est construit, par ligature ou sous forme de plasmide. Cet ADN est ensuite utilisé pour générer un premier virus recombinant portant lesdites délétions et un ADN recombinant. L'ADN de ce premier virus est ensuite isolé et co-transfecté avec un second plasmide ou l'ADN d'un second virus recombinant défectif portant le deuxième ADN recombinant, les délétions appropriées (partie non présente sur le premier virus), et une région permettant la recombinaison homologue. Cette deuxième étape génère ainsi le virus recombinant défectif portant les deux ADN recombinants. Cette variante de préparation est particulièrement appropriée pour la préparation de virus recombinants portant deux ADN recombinants insérés en deux régions différentes du génome de l'adénovirus.

Les deux agents selon l'invention à savoir l'immunosuppresseur et l'adénovirus recombinant peuvent être formulés en vue d'une administration par voie topique, orale, parentérale, intranasale, intraveineuse, intramusculaire, sous-cutanée, intraoculaire, transdermique, etc.

Préférentiellement, la ou les formulations pharmaceutiques respectives contiennent des véhicules pharmaceutiquement acceptables pour une formulation injectable. Il peut s'agir en particulier de solutions salines (phosphate monosodique, disodique, chlorure de sodium, potassium, calcium ou magnésium, etc, ou des mélanges de tels sels), stériles, isotoniques, ou de compositions sèches, notamment lyophilisées, qui, par addition selon le cas d'eau stérilisée ou de sérum physiologique, permettent la constitution de solutés injectables.

Les doses en immunosuppresseur et en adénovirus utilisées pour l'injection peuvent être adaptées en fonction de différents paramètres, et notamment en fonction du mode d'administration utilisé, de la pathologie concernée, du gène à exprimer, ou encore de la durée du traitement recherchée.

D'une manière générale, les adénovirus recombinants selon l'invention sont formulés et administrés sous forme de doses comprises entre 10⁴ et 10¹⁴ pfu/ml, et de préférence 10⁶ à 10¹⁰ pfu/ml. Le terme pfu ("plaque forming unit") correspond au pouvoir infectieux d'une solution considérée, et est déterminé par infection d'une culture cellulaire appropriée, et mesure, généralement après 5 jours, du nombre de plages de cellules infectées. Les techniques de détermination du titre pfu d'une solution virale sont bien documentées dans la littérature. En ce qui concerne plus particulièrement les immunosuppresseurs, leurs doses et modes d'injection varient selon leur nature. L'ajustement de ces deux paramètres entrent dans les compétences de l'homme du métier.

L'association médicamenteuse selon les revendications 1 à 25 peut être utilisée pour le traitement ou la prévention de nombreuses pathologies. Selon le gène thérapeutique inséré dans son adénovirus, elle peut être utilisée notamment pour le traitement ou la prévention des maladies génétiques (dystrophie, muscoviscidose, etc), des maladies neurodégénératives (alzheimer, parkinson, ALS, etc), des pathologies hyperprolifératives (cancers, resténose, etc), des pathologies liées aux désordres de la coagulation ou aux dyslipoprotéinémies, des pathologies liées aux infections virales (hépatites, SIDA, etc), etc.

La présente invention sera plus complètement décrite à l'aide des exemples qui suivent, qui doivent être considérés comme illustratifs et non limitatifs.

Figure 1 : Organisation génétique de l'adénovirus Ad5. La séquence complète de l'Ad5 est disponible sur base de données et permet à l'homme du métier de sélectionner ou de créer tout site de restriction, et ainsi d'isoler toute région du génome.

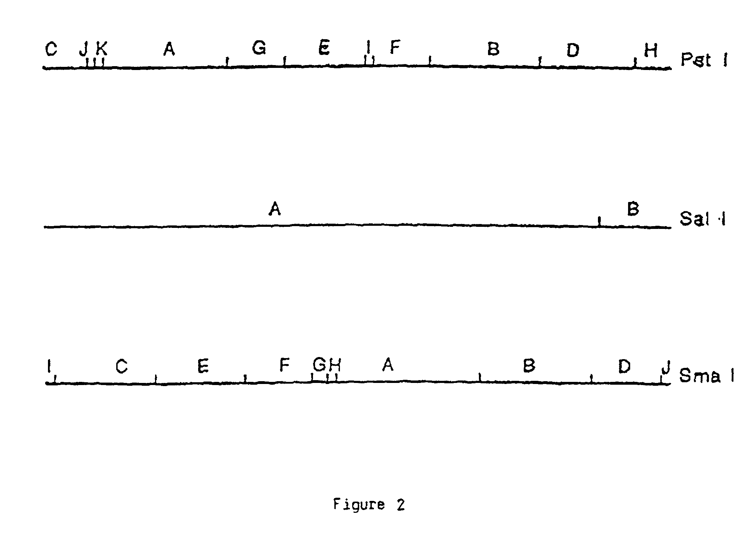

Figure 2 : Carte de restriction de l'adénovirus CAV2 souche Manhattan (d'après Spibey et al précité).

Figure 3 : Construction du vecteur pAD5-gp19k-ßgal.

Figure 4 : Construction de l'adénovirus Ad-gp19k-ßgal,ΔE1,ΔE3.

### Techniques générales de biologie moléculaire

Les méthodes classiquement utilisées en biologie moléculaire telles que les extractions préparatives d'ADN plasmidique, la centrifugation d'ADN plasmidique en gradient de chlorure de césium, l'électrophorèse sur gels d'agarose ou d'acrylamide, la purification de fragments d'ADN par électroélution, les extraction de protéines au phénol ou au phénol-chloroforme, la précipitation d'ADN en milieu salin par de l'éthanol ou de l'isopropanol, la transformation dans Escherichia coli, etc ... sont bien connues de l'homme de métier et sont abondament décrites dans la littérature [Maniatis T. et al., "Molecular Cloning, a Laboratory Manual", Cold Spring Harbor Laboratory, Cold Spring Harbor, N.Y., 1982; Ausubel F.M. et al. (eds), "Current Protocols in Molecular Biology", John Wiley & Sons, New York, 1987].
Les plasmides de type pBR322, pUC et les pliages de la série M13 sont d'origine commerciale (Bethesda Research Laboratories).
Pour les ligatures, les fragments d'ADN peuvent être séparés selon leur taille par électrophorèse en gels d'agarose ou d'acrylamide, extraits au phénol ou par un mélange phénol/chloroforme, précipités à l'éthanol puis incubés en présence de l'ADN ligase du phage T4 (Biolabs) selon les recommandations du fournisseur.
Le remplissage des extrémités 5' proéminentes peut être effectué par le fragment de Klenow de l'ADN Polymérase I d'E. coli (Biolabs) selon les spécifications du fournisseur. La destruction des extrémités 3' proéminentes est effectuée en présence de l'ADN Polymérase du phage T4 (Biolabs) utilisée selon les recommandations du fabricant. La destruction des extrémités 5' proéminentes est effectuée par un traitement ménagé par la nucléase S1.
La mutagénèse dirigée in vitro par oligodéoxynucléotides synthétiques peut être effectuée selon la méthode développée par Taylor et al. [Nucleic Acids Res. 13 (1985) 8749-8764] en utilisant le kit distribué par Amersham.
L'amplification enzymatique de fragments d'ADN par la technique dite de PCR [Polymérase-catalyzed Chain Reaction, Saiki R.K. et al., Science 230 (1985) 1350-1354; Mullis K.B. et Faloona F.A., Meth. Enzym. 155 (1987) 335-350] peut être effectuée en utilisant un "DNA thermal cycler" (Perkin Elmer Cetus) selon les spécifications du fabricant.
La vérification des séquences nucléotidiques peut être effectuée par la méthode développée par Sanger et al. [Proc. Natl. Acad. Sci. USA, 74 (1977) 5463-5467] en utilisant le kit distribué par Amersham.

### Lignées cellulaires utilisées

Dans les exemples qui suivent, les lignées cellulaires suivantes ont ou peuvent être utilisées :
- Lignée de rein embryonnaire humain 293 (Graham et al., J. Gen. Virol. 36 (1977) 59). Cette lignée contient notamment, intégrée dans son génome, la partie gauche du génome de l'adénovirus humain Ad5 (12 %).
- Lignée de cellules humaines KB : Issue d'un carcinome épidermique humain, cette lignée est accessible à l'ATCC (ref. CCL17) ainsi que les conditions permettant sa culture.
- Lignée de cellules humaines Héla : Issue d'un carcinome de l'épithelium humain, cette lignée est accessible à l'ATCC (ref. CCL2) ainsi que les conditions permettant sa culture.
- Lignée de cellules canines MDCK : Les conditions de culture des cellules MDCK ont été décrites notamment par Macatney et al., Science 44 (1988) 9.
- Lignée de cellules gm DBP6 (Brough et al., Virology 190 (1992) 624). Cette lignée est constituée de cellules Hela portant le gène E2 d'adénovirus sous le controle du LTR de MMTV.

### EXEMPLES

### Exemple 1. Construction d'adénovirus recombinants défectifs comprenant un gène thérapeutique (le gène LacZ de E. coli) sous le contrôle du promoteur du LTR du RSV et le gène gp19k sous le contrôle du promoteur du LTR du RSV, tous deux insérés au niveau de la région E1.

Ces adénovirus ont été construits par recombinaison homologue entre un plasmide portant la partie gauche de l'adénovirus Ad5, les deux ADN recombinants et une région de l'adénovirus Ad5 (correspondant à la protéine IX) et l'ADN d'un adénovirus défectif portant différentes délétions.

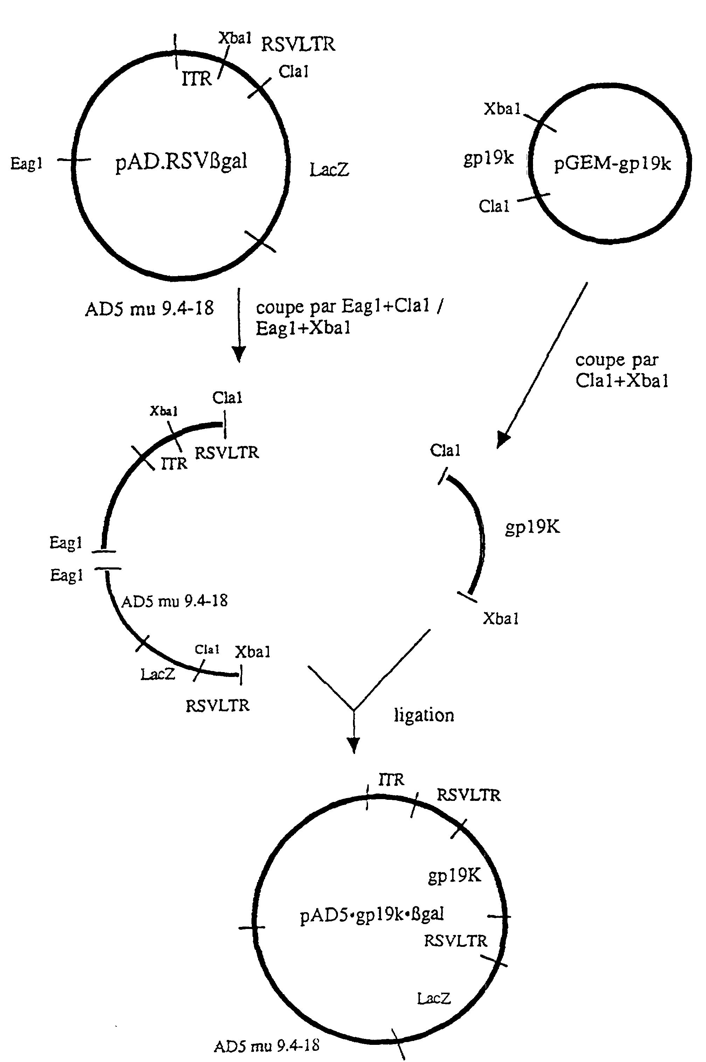

### 1. Construction du vecteur pAD5-gpl9k-βgal (figure 3)

### 1.1. Construction du plasmide pGEM-gp19k

Le plasmide pAD5-gp19k-βgal contient une séquence d'ADNc codant pour la protéine gp19k d'adénovirus. Ce plasmide a été construit comme suit. Le fragment XbaI du génome de l'adénovirus Ad5 sauvage contenant la région E3 a été isolé et cloné au site correspondant du plasmide pGEM (Promega) pour générer le plasmide pGEM-E3. Le fragment HinfI contenant la séquence codante de la gp19k (nucléotides 28628 à 29634 de l'adénovirus Ad5 sauvage) a ensuite été isolé à partir du plasmide pGEM-E3. Les extrémités de ce fragment ont été rendues franches par action du fragment de Klenow de l'ADN polymérase I d'E. coli (Cf techniques générales de biologie moléculaire), puis le fragment obtenu a été cloné au site SmaI du plasmide pGEMzf+ (Promega).

Le plasmide obtenu a été désigné pGEM-gp19k (figure 3).

### 1.2. Construction du vecteur pAD5-gp19k-βgal

Cet exemple décrit la construction d'un plasmide contenant un les deux ADN recombinants comprenant leur propre promoteur, la partie gauche du génome de l'adénovirus et une partie supplémentaire (protéine pIX) permettant la recombinaison homologue. Ce vecteur a été construit à partir du plasmide pAd.RSVβGal comme suit

Le plasmide pAd.RSVβGal contient, dans l'orientation 5'->3',
- le fragment PvuII correspondant à l'extrémité gauche de l'adénovirus Ad5 comprenant : la séquence ITR, l'origine de réplication, les signaux d'encapsidation et l'amplificateur E1A;
- le gène codant pour la β-galactosidase sous le contrôle du promoteur RSV (du virus du sarcome de Rous),
- un second fragment du génome de l'adénovirus Ad5, qui permet la recombinaison homologue entre le plasmide pAd.RSVβGal et l'adénovirus d1324. Le plasmide pAd.RSVβGal a été décrit par Stratford-Perricaudet et al. (J. Clin. Invest. 90 (1992) 626).

Le plasmide pAd.RSVβGal a tout d'abord été coupé par les enzymes EagI et ClaI. Ceci génère un premier fragment portant notamment la partie gauche de l'adénovirus Ad5 et le promoteur du LTR du RSV. Parallèlement, le plasmide pAd.RSVβGal a également été coupé par les enzymes EagI et XbaI. Ceci génère un deuxième type de fragment portant notamment le promoteur du LTR du RSV, le gène LacZ, et un fragment du génome de l'adénovirus Ad5, qui permet la recombinaison homologue. Les fragments ClaI-EagI et EagI-XbaI ont ensuite été ligaturés en présence du fragment XbaI-ClaI du plasmide pGEM-gp19k (exemple 1.1) portant la séquence codante de la gp19k (Cf figure 3). Le vecteur ainsi obtenu, désigné pAD5-gp19k-βgal, contient donc
- le fragment PvuII correspondant à l'extrémité gauche de l'adénovirus Ad5 comprenant : la séquence ITR, l'origine de réplication, les signaux d'encapsidation et l'amplificateur E1A;
- la séquence codant pour la gp19k sous le contrôle du promoteur RSV (du virus du sarcome de Rous);
- le gène codant pour la β-galactosidase sous le contrôle du promoteur RSV (du virus du sarcome de Rous), et,
- un second fragment du génome de l'adénovirus Ad5, qui permet la recombinaison homologue.

### 2. Construction des adénovirus recombinants

### 2.1. Construction d'un adénovirus recombinant délété dans la région E1, portant les deux ADN recombinants insérés dans la même orientation, au niveau de la région E1.

Le vecteur pAD5-gp19k-βgal a été linéarisé et cotransfecté avec un vecteur adénoviral déficient dans le gène E1, dans les cellules helper (lignée 293) apportant en *trans* les fonctions codées par les régions E1 (E1A et E1B) d'adénovirus.
Plus précisément, l'adénovirus Ad-gp19k-βgal,ΔE1 est obtenu par recombinaison homologue in vivo entre l'adénovirus Ad-RSVβgal (Cf Stratford-Perricaudet et al citée plus haut) et le vecteur pAD5-gp19k-βgal, selon le protocole suivant : le plasmide pAD5-gp19k-βgal, linéarisé par XmnI, et l'adénovirus Ad-RSVβgal, linéarisé par l'enzyme ClaI, sont co-transfectés dans la lignée 293 en présence de phosphate de calcium, pour permettre la recombinaison homologue. Les adénovirus recombinants ainsi générés sont ensuite sélectionnés par purification sur plaque. Après isolement, l'ADN de l'adénovirus recombinant est amplifié dans la lignée cellulaire 293, ce qui conduit à un surnageant de culture contenant l'adénovirus défectif recombinant non purifié ayant un titre d'environ 10¹⁰ pfu/ml.
Les particules virales sont généralement purifiées par centrifugation sur gradient de chlorure de césium selon les techniques connues (voir notamment Graham et al., Virology 52 (1973) 456). L'adénovirus Ad-gp19k-βgal,ΔE1 peut être conservé à -80°C dans 20 % de glycérol.

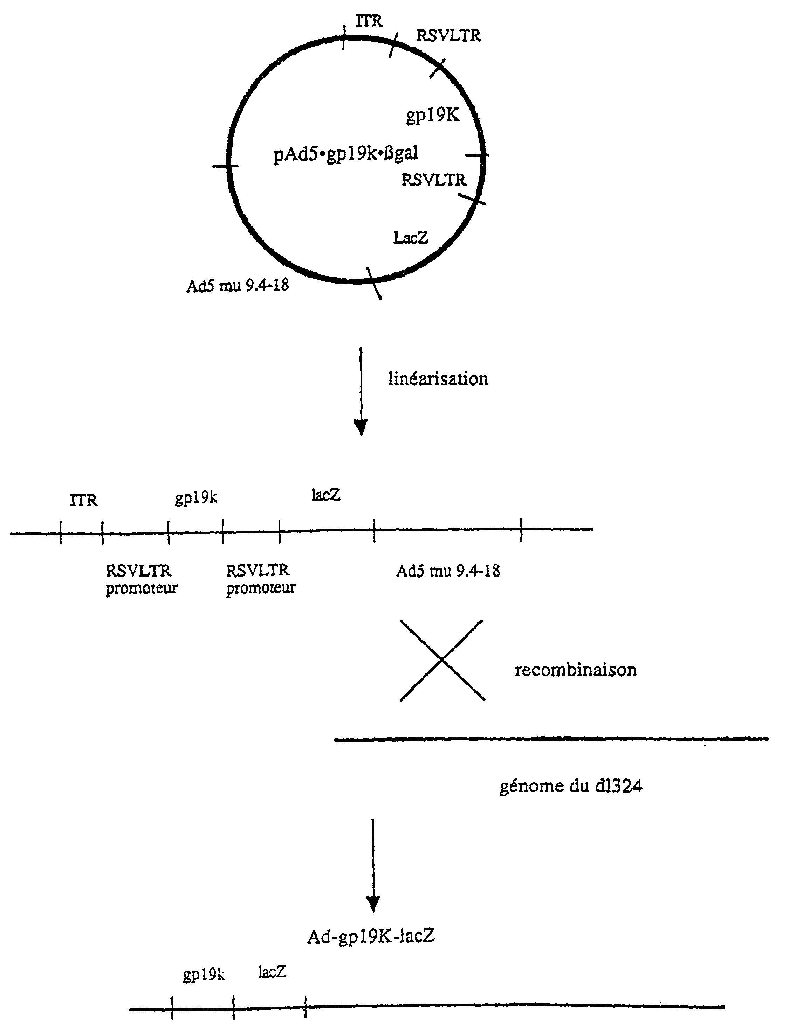

### 2.2. Construction d'un adénovirus recombinant délété dans les régions E1 et E3, portant les deux ADN recombinants insérés dans la même orientation, au niveau de la région E1 (figure 4).

Le vecteur pAD5-gp19k-βgal a été linéarisé et cotransfecté avec un vecteur adénoviral déficient dans les gènes E1 et E3, dans les cellules helper (lignée 293) apportant en *trans* les fonctions codées par les régions E1 (E1A et E1B) d'adénovirus.
Plus précisément, l'adénovirus Ad-gp19k-βgal,ΔE1,ΔE3 a été obtenu par recombinaison homologue in vivo entre l'adénovirus mutant Ad-dl1324 (Thimmappaya et al., Cell 31 (1982) 543) et le vecteur pAD5-gp19k-βgal, selon le protocole suivant : le plasmide pAD5-gp19k-βgal et l'adénovirus Ad-dl1324, linéarisé par l'enzyme ClaI, ont été co-transfectés dans la lignée 293 en présence de phosphate de calcium, pour permettre la recombinaison homologue. Les adénovirus recombinants ainsi générés ont ensuite été sélectionnés par purification sur plaque. Après isolement, l'ADN de l'adénovirus recombinant est amplifié dans la lignée cellulaire 293, ce qui conduit à un surnageant de culture contenant l'adénovirus défectif recombinant non purifié ayant un titre d'environ 10¹⁰ pfu/ml.
Les particules virales sont généralement purifiées par centrifugation sur gradient de chlorure de césium selon les techniques connues (voir notamment Graham et al., Virology 52 (1973) 456). Le génome de l'adénovirus recombinant a ensuite été vérifié par analyse en southern blot. L'adénovirus Ad-gp19k-βgal,ΔE1,ΔE3 peut être conservé à -80°C dans 20 % de glycérol.

### Exemple 2 : Mise en évidence de l'activité immunoprotectrice de l'association médicamenteuse selon l'invention.

60 souris adultes femelles DBA/2 sont réparties au hasard en 6 groupes de 10 souris traités respectivement selon les protocoles d'injection suivants :
- **GROUPE 1a:**
   Il reçoit une injection intraoculaire de 10 µg d'anticorps monoclonaux anti-CD3 aux jours -2, -1, 1, 2, 3, 4 et 5 avec au jour 0 une injection intraveineuse de 4.10⁹ pfu de virus Ad-RSVβgal (Cf Stratford-Perricaudet et al citée plus haut).
- **GROUPE 1b:**
   Il reçoit le même traitement que le groupe la en utilisant à titre de virus, 4.10⁹ pfu du virus Ad-gp 19 k-βgal ( figure 4).
- **GROUPE 2a:**
   Il reçoit une injection intrapétitonéale de 250 µg d'anticorps monoclonaux anti-CD4 aux jours -2,-1,1,4, 7 avec au jour 0 une injection intraveineuse de 4.10⁹ pfu du virus Ad-RSV β gal.
- **GROUPE 2b:**
   Il reçoit le même traitement que le groupe 2a en utilisant à titre de virus, 4.10⁹ pfu du virus Ad gp 19k-β gal.
- **GROUPE 3a:**
   Il reçoit une injection intraveineuse de 4.10⁹ pfu d'Ad-βgal sans administration conjointe d'immunosuppresseur.
- **GROUPE 3b:**
   Il reçoit une injection intraveineuse de 4.10⁹ pfu d'Ad-gp19k-βgal sans administration conjointe d'immunosuppresseur.

A différents temps, deux animaux de chaque groupe ont été sacrifiés dans le but de prélever le foie et la rate.

### 2.1. - Analyse par immunofluorescence de la répartition des principales sous-populations lymphocytaires (CD3+, CD4+ et CD8+) au sein des splénocytes prélevés à J15 après l'injection.

Une suspension de cellules isolées a été préparée à partir des rates prélevées. Un échantillon de cellules a été analysé par immunofluorescence, à l'aide d'anticorps spécifiques de chaque sous-population lymphocytaire. La lecture des cellules fluorescentes a été réalisée grâce à un cytofluoromètre (FACS Schan-Becton Dickinson). Le tableau I qui suit rend compte des résultats.

**TABLEAU I**

| | Groupe 3 a Ad-βgal | | Groupe 3b Ad-βgal-gp19k | | Groupe 1a anti CD3/ Ad-βgal | | Groupe 2a anti CD4/ Ad-βgal | |
|---|---|---|---|---|---|---|---|---|
| | % de cellules exprimant βgal à la surface cellulaire | | | | | | | |
| **CD3** | 20,0 | 17,5 | 20,6 | 21 | 5,4 | 6,1 | 12 | 10,3 |
| **CD4** | 13,4 | 12,6 | 15,3 | 16,8 | 4,4 | 5,1 | 2,7 | 4,1 |
| **CD8** | 5,5 | 5,5 | 6,1 | 6 | 2,02 | 2,3 | 7,9 | 6,6 |

On note la nette diminution des cellules CD3+ CD4+ et CD8+ chez les animaux traités par anti-CD3 et la diminution sélective des cellules CD4+ chez les animaux traités par anti-CD4

### 2.2- Analyse de la capacité cytotoxique des splénocytes prélevés à J32 après l'injection et stimulés in vitro vis-à-vis de cibles histocompatibles exprimant la βgal

Un deuxième échantillon de splénocytes isolés à partir des rates des animaux traités a été cultivé in vitro pendant 4 jours en présence de cellules P815 exprimant la βgalactosidase à leur surface. Au terme de la culture, l'activité cytotoxique des splénocytes vis-à-vis de cibles P815-βgal marquées au Cr⁵¹ a été évaluée. L'activité cytotoxique, exprimée par le pourcentage de cytolyse, a été déterminée de manière conventionnelle en mettant en présence différents rapports de cellules effectrices et de cellules cibles. Les résultats sont présentés dans le tableau II ci-après.

**TABLEAU II**

| Groupe | 2b | 3A |
|---|---|---|
| Traitement | Anti-CD4/ Ad-βgal-gp19k | AD-βgal |
| Rapport Effecteur/cibles | % de cytolyse | |
| 80/1 | 4 2 | 13 14 |
| 40/1 | 2 1 | 13 9 |
| 20/1 | 1 1 | 5 9 |
| 10/1 | 0 0 | 2 5 |
| 5/1 | 0 1 | 1 2 |
| Les splénocytes, prélevés chez les animaux ayant reçu le traitement par anti CD4 c'est-à-dire le groupe 2b, montrent une neutralisation très nette de leur capacité cytotoxique. | | |

### 2.3 Expression de l'activité βgalactosidase dans le foie après 15 et 32 jours.

Les foies sont sectionnés et colorés avec du X-gal pour révéler l'activité β galactosidase et de l'éosine pour mettre en évidence l'histologie de la section. Les résultats sont présentés dans le tableau III² ci-après.

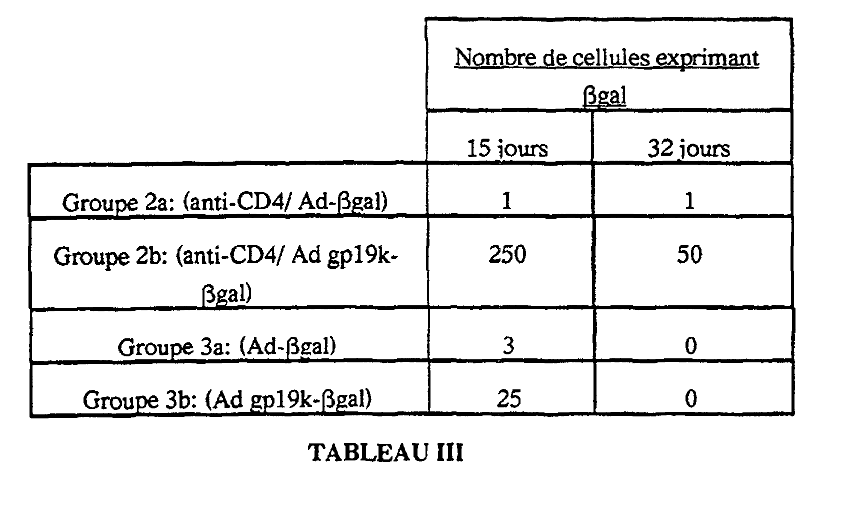

Des résultats présentés ci-dessus, il ressort que l'injection d'anticorps anti-CD4 associée à une injection d'Adgp19k-βgal induit une expression nettement prolongée du gène considéré. C'est ainsi que 30 jours après les injections, on observe dans le cas du groupe 2b une activité β galactosidase significative. Cette prolongation qui peut être interprétée comme la conséquence d'un phénomène de tolérance induit selon l'invention est nettement supérieure à celle qui pouvait être attendue de la simple juxtaposition des effets respectifs de l'immunosuppresseur anti-CD4 et de l'adénovirus recombinant Ad gp19k -βgal.
Aucune réaction inflammatoire n'est en outre observée dans ce délai de 30 jours dans le cas du groupe 2b.

## Revendications

1. Association médicamenteuse d'au moins un agent immunosuppresseur et d'au moins un adénovirus recombinant défectif dont le génome comprend un premier ADN recombinant contenant un gène thérapeutique et un second ADN recombinant contenant un gène immunoprotecteur, pour une utilisation consécutive, intermittente et/ou simultanée dans le temps, utile pour des transfections exogéniques in vivo et/ou ex-vivo.

2. Association médicamenteuse selon la revendication 1 caractérisée en ce que l'agent immunosuppresseur est de préférence choisi parmi la cyclosporine, le FK506, l'azathioprine, les corticostéroïdes et des anticorps mono- ou poly-clonaux.

3. Association médicamenteuse selon la revendication 2 caractérisée en ce qu'il s'agit d'anticorps capables d'inactiver des molécules immunes ou de provoquer la destruction des cellules immunes portant ces molécules .

4. Association médicamenteuse selon la revendication 3 caractérisée en ce que l'anticorps est choisi parmi les anti-CD4, -CD2, -CD3, -CD8, -CD28, -B7,-ICAM-1, -LFA-1 et la CTLA4Ig.

5. Association médicamenteuse selon l'une des revendications précédentes caractérisée en ce que le gène thérapeutique code pour une protéine thérapeutique.

6. Association médicamenteuse selon l'une des revendications 1 à 4 caractérisée en ce que le gène thérapeutique code pour un ARN thérapeutique.

7. Association médicamenteuse selon l'une des revendications précédentes caractérisée en ce que le gène immunoprotecteur est un gène dont le produit agit sur l'activité du complexe majeur d'histocompatibilité (MHC) ou sur l'activité des cytokines.

8. Association médicamenteuse selon la revendication 7 caractérisée en ce que le gène immunoprotecteur est un gène dont le produit inhibe au moins partiellement l'expression des protéines du MHC ou la présentation antigénique.

9. Association médicamenteuse selon l'une des revendications précédentes caractérisée en ce que le gène immunoprotecteur est choisi parmi le gène de la gp19k de l'adénovirus, le gène ICP47 du virus de l'herpès, ou le gène UL18 du cytomégalovirus.

10. Association médicamenteuse selon l'une des revendications précédentes caractérisée en ce que les deux ADN recombinants du génome de l'adénovirus constituent une entité transcriptionnelle unique.

11. Association médicamenteuse selon l'une des revendications 1 à 9 caractérisée en ce que les deux ADN recombinants comportent chacun un promoteur transcriptionnel, identique ou différent.

12. Association médicamenteuse selon la revendication 11 caractérisée en ce que les deux ADN recombinants sont insérés dans la même orientation.

13. Association médicamenteuse selon la revendication 11 caractérisée en ce que les deux ADN recombinants sont insérés dans les orientations opposées.

14. Association médicamenteuse selon l'une des revendications précédentes caractérisée en ce que les deux ADN recombinants sont insérés dans un même site du génome de l'adénovirus, de préférence au niveau des régions E1, E3 ou E4.

15. Association médicamenteuse selon la revendication 14 caractérisée en ce que les deux ADN recombinants sont insérés au niveau de la région E1.

16. Association médicamenteuse selon l'une des revendications 1 à 13 caractérisée en ce que les deux ADN recombinants sont insérés en des sites différents du génome de l'adénovirus.

17. Association médicamenteuse selon la revendication 16 caractérisée en ce que l'un des ADN recombinants est inséré au niveau de la région E1 et l'autre au niveau de la région E3 ou E4.

18. Association médicamenteuse selon l'une des revendications précédentes caractérisée en ce que l'adénovirus est un adénovirus recombinant défectif comprenant les séquences ITR, une séquence permettant l'encapsidation, et portant une délétion de tout ou partie des gènes E1 et E4.

19. Association médicamenteuse selon la revendication 18 caractérisée en ce qu'il s'agit d'un adénovirus comprenant les séquences ITR, une séquence permettant l'encapsidation et portant une délétion de tout ou partie des gènes E1, E3 et E4.

20. Association médicamenteuse selon l'une des revendications 1 à 19 caractérisée en ce qu'il s'agit d'un adénovirus dont le génome est délété de tout ou partie des gènes E1, E3, L5 et E4.

21. Association médicamenteuse selon l'une des revendications précédentes caractérisée en ce que l'adénovirus recombinant est d'origine humaine, animale, ou mixte.

22. Association médicamenteuse selon la revendication 21 caractérisée en ce que les adénovirus recombinants d'origine humaine sont choisis parmi ceux classés dans le groupe C, de préférence parmi les adénovirus recombinants de type 2 ou 5 (Ad 2 ou Ad 5).

23. Association médicamenteuse selon la revendication 21 caractérisée en ce que les adénovirus d'origine animale sont choisis parmi les adénovirus d'origine canine, bovine, murine, ovine, porcine, aviaire et simienne.

24. Association médicamenteuse selon l'une des revendications précédentes caractérisée en ce que l'agent immunosuppresseur est injecté avant et après l'injection de l'adénovirus.

25. Association médicamenteuse selon l'une des revendications 1 à 23 caractérisée en ce que l'agent immunosuppresseur et l'adénovirus recombinant sont injectées simultannément.

## Claims

1. Medicinal combination of at least one immunosuppressive agent and of at least one defective recombinant adenovirus, the genome of which comprises a first recombinant DNA containing a therapeutic gene and a second recombinant DNA containing an immunoprotective gene, for use which is consecutive, intermittent and/or simultaneous in time, which can be used for exogenic transfections in vivo and/or ex vivo.

2. Medicinal combination according to Claim 1, characterized in that the immunosuppressive agent is preferably chosen from cyclosporin, FK506, azathioprine, corticosteroids and mono- or polyclonal antibodies.

3. Medicinal combination according to Claim 2, characterized in that the antibodies are antibodies capable of inactivating immune molecules or of causing the destruction of the immune cells carrying these molecules.

4. Medicinal combination according to Claim 3, characterized in that the antibody is chosen from anti-CD4, -CD2, -CD3, -CD8, -CD28, -B7, -ICAM-1 and -LFA-1 antibodies and CTLA4Ig.

5. Medicinal combination according to one of the preceding claims, characterized in that the therapeutic gene encodes a therapeutic protein.

6. Medicinal combination according to one of Claims 1 to 4, characterized in that the therapeutic gene encodes a therapeutic RNA.

7. Medicinal combination according to one of the preceding claims, characterized in that the immunoprotective gene is a gene the product of which acts on the activity of the major histocompatibility complex (MHC) or on the activity of cytokines.

8. Medicinal combination according to Claim 7, characterized in that the immunoprotective gene is a gene the product of which inhibits, at least partially, the expression of MHC proteins or antigen presentation.

9. Medicinal combination according to one of the preceding claims, characterized in that the immunoprotective gene is chosen from the adenovirus gp19k gene, the herpesvirus ICP47 gene or the cytomegalovirus UL18 gene.

10. Medicinal combination according to one of the preceding claims, characterized in that the two recombinant DNAs of the adenovirus genome constitute a single transcriptional entity.

11. Medicinal combination according to one of Claims 1 to 9, characterized in that the two recombinant DNAs each comprise a transcriptional promoter, which may be identical or different.

12. Medicinal combination according to Claim 11, characterized in that the two recombinant DNAs are inserted in the same direction.

13. Medicinal combination according to Claim 11, characterized in that the two recombinant DNAs are inserted in opposite directions.

14. Medicinal combination according to one of the preceding claims, characterized in that the two recombinant DNAs are inserted into the same site of the adenovirus genome, preferably into the E1, E3 or E4 regions.

15. Medicinal combination according to Claim 14, characterized in that the two recombinant DNAs are inserted into the E1 region.

16. Medicinal combination according to one of Claims 1 to 13, characterized in that the two recombinant DNAs are inserted into different sites of the adenovirus genome.

17. Medicinal combination according to Claim 16, characterized in that one of the recombinant DNAs is inserted into the E1 region and the other into the E3 or E4 region.

18. Medicinal combination according to one of the preceding claims, characterized in that the adenovirus is a defective recombinant adenovirus comprising the ITR sequences and a sequence allowing the encapsidation, and carrying a deletion of all or part of the E1 and E4 genes.

19. Medicinal combination according to Claim 18, characterized in that the adenovirus is an adenovirus comprising the ITR sequences and a sequence allowing the encapsidation, and carrying a deletion of all or part of the E1, E3 and E4 genes.

20. Medicinal combination according to one of Claims 1 to 19, characterized in that the adenovirus is an adenovirus the genome of which is deleted of all or part of the E1, E3, L5 and E4 genes.

21. Medicinal combination according to one of the preceding claims, characterized in that the recombinant adenovirus is of human, animal or mixed origin.

22. Medicinal combination according to Claim 21, characterized in that the recombinant adenoviruses of human origin are chosen from those classified in group C, preferably from the type 2 or 5 (Ad 2 or Ad 5) recombinant adenoviruses.

23. Medicinal combination according to Claim 21, characterized in that the adenoviruses of animal origin are chosen from the adenoviruses of canine, bovine, murine, ovine, porcine, avian and simian origin.

24. Medicinal combination according to one of the preceding claims, characterized in that the immunosuppressive agent is injected before and after the injection of the adenovirus.

25. Medicinal combination according to one of Claims 1 to 23, characterized in that the immunosuppressive agent and the recombinant adenovirus are injected simultaneously.

## Patentansprüche

1. Medikamentöse Assoziation mindestens eines immunsuppressiven Mittels und mindestens eines defekten rekombinanten Adenovirus, dessen Genom eine erste rekombinante DNA, die ein therapeutisches Gen enthält, und eine zweite rekombinante DNA, die ein immunschützendes Gen enthält, umfasst, zur aufeinanderfolgenden, zeitlich abgestuften und/oder gleichzeitigen Verwendung für exogene in vivo- und/oder ex-vivo-Transfektionen.

2. Medikamentöse Assoziation nach Anspruch 1, dadurch **gekennzeichnet** , dass das immunsuppressive Mittel bevorzugt aus Cyclosporin, FK506, Azathioprin, Corticosteroiden und mono- oder polyclonalen Antikörpern ausgewählt ist.

3. Medikamentöse Assoziation nach Anspruch 2, dadurch **gekennzeichnet** , dass es sich um Antikörper mit der Fähigkeit, Immunmoleküle zu inaktivieren oder Zerstörung von Immunzellen, die diese Moleküle tragen, hervorzurufen, handelt.

4. Medikamentöse Assoziation nach Anspruch 3, dadurch **gekennzeichnet** , dass der Antikörper aus anti-CD4, -CD2, -CD3, -CD8, -CD28, -B7, -ICAM-1, -LFA-1 und CTLA4Ig ausgewählt ist.

5. Medikamentöse Assoziation nach einem der vorstehenden Ansprüche dadurch **gekennzeichnet** , dass das therapeutische Gen ein therapeutisches Protein codiert.

6. Medikamentöse Assoziation nach einem der Ansprüche 1 bis 4, dadurch **gekennzeichnet** , dass das therapeutische Gen eine therapeutische RNA codiert.

7. Medikamentöse Assoziation nach einem der vorstehenden Ansprüche, dadurch **gekennzeichnet,** dass das immunschützende Gen ein Gen ist, dessen Produkt auf die Aktivität des Haupt-Histokompatibilitäts-Komplexes (MHC) oder die Aktivität der Cytokine einwirkt.

8. Medikamentöse Assoziation nach Anspruch 7, dadurch **gekennzeichnet,** dass das immunschützende Gen ein Gen ist, dessen Produkt mindestens teilweise die Expression der MHC-Proteine oder der Antigenpräsentation hemmt.

9. Medikamentöse Assoziation nach einem der vorstehenden Ansprüche, dadurch **gekennzeichnet,** dass das immunschützende Gen aus dem Gen von gp19k des Adenovirus, dem Gen ICP47 des Herpesvirus oder dem Gen UL18 des Cytomegalievirus ausgewählt ist.

10. Medikamentöse Assoziation nach einem der vorstehenden Ansprüche, dadurch **gekennzeichnet** , dass die zwei rekombinanten DNAs des Genoms des Adenovirus eine einzige Transkriptionseinheit darstellen.

11. Medikamentöse Assoziation nach einem der Ansprüche 1 bis 9, dadurch **gekennzeichnet,** dass die zwei rekombinanten DNAs jeweils einen gleichen oder unterschiedlichen Transkriptionspromotor umfassen.

12. Medikamentöse Assoziation nach Anspruch 11, dadurch **gekennzeichnet,** dass die zwei rekombinanten DNAs in gleicher Orientierung insertiert sind.

13. Medikamentöse Assoziation nach Anspruch 11, dadurch **gekennzeichnet,** dass die zwei rekombinanten DNAs in entgegengesetzten Orientierungen insertiert sind.

14. Medikamentöse Assoziation nach einem der vorstehenden Ansprüche, dadurch **gekennzeichnet,** dass die zwei rekombinanten DNAs an der gleichen Stelle des Genoms des Adenovirus, bevorzugt in den Regionen E1, E3 oder E4, insertiert sind.

15. Medikamentöse Assoziation nach Anspruch 14, dadurch **gekennzeichnet,** dass die zwei rekombinanten DNAs in der Region E1 insertiert sind.

16. Medikamentöse Assoziation nach einem der Ansprüche 1 bis 13, dadurch **gekennzeichnet,** dass die zwei rekombinanten DNAs an verschiedenen Stellen des Genoms des Adenovirus insertiert sind.

17. Medikamentöse Assoziation nach Anspruch 16, dadurch **gekennzeichnet,** dass die eine der rekombinanten DNAs in der Region E1 und die andere in der Region E3 oder E4 insertiert ist.

18. Medikamentöse Assoziation nach einem der vorstehenden Ansprüche, dadurch **gekennzeichnet,** dass das Adenovirus ein defektes rekombinantes Adenovirus, umfassend die Sequenzen ITR, eine Sequenz, die die Verkapselung erlaubt, und enthaltend eine teilweise oder vollständige Deletion der Gene E1 und E4, ist.

19. Medikamentöse Assoziation nach Anspruch 18, dadurch **gekennzeichnet,** dass es sich um ein Adenovirus handelt, das die ITR-Sequenzen, eine Sequenz, die die Verkapselung erlaubt und eine ganze oder teilweise Deletion der Gene E1, E3 und E4 enthält, umfasst.

20. Medikamentöse Assoziation nach einem der Ansprüche 1 bis 19, dadurch **gekennzeichnet,** dass es sich um ein Adenovirus handelt, dessen Genom ganz oder teilweise in den Genen E1, E3, L5 und E4 deletiert ist.

21. Medikamentöse Assoziation nach einem der vorstehenden Ansprüche, dadurch **gekennzeichnet,** dass das rekombinante Adenovirus menschlichen, tierischen oder gemischten Ursprungs ist.

22. Medikamentöse Assoziation nach Anspruch 21, dadurch **gekennzeichnet,** dass die rekombinanten Adenoviren menschlichen Ursprungs aus denjenigen, die in die Gruppe C klassifiziert sind, bevorzugt aus den rekombinanten Adenoviren vom Typ 2 oder 5 (Ad 2 oder Ad 5), ausgewählt sind.

23. Medikamentöse Assoziation nach Anspruch 21, dadurch **gekennzeichnet,** dass die Adenoviren tierischen Ursprungs aus Adenoviren aus Hund, Rind, Maus, Schaf, Schwein, Vogel und Affe ausgewählt sind.

24. Medikamentöse Assoziation nach einem der vorstehenden Ansprüche, dadurch **gekennzeichnet,** dass das immunsuppressive Mittel vor und nach der Injektion des Adenovirus injiziert wird.

25. Medikamentöse Assoziation nach einem der Ansprüche 1 bis 23, dadurch **gekennzeichnet,** dass das immunsuppressive Mittel und das rekombinante Adenovirus gleichzeitig injiziert werden.
